# EUROPEAN PATENT SPECIFICATION

(11) **EP 3 020 448 B1**
(45) Date of publication and mention of the grant of the patent: **06.09.2023**
(21) Application number: 14823684.7
(22) Date of filing: 30.06.2014
(51) Int. Cl.: A61N 1/36, A61N 1/04, A61H 19/00

(54) **PERCUTANEOUS STIMULATOR FOR REDUCING SENSITIVITY OF NERVES**
PERKUTANER STIMULATOR ZUR REDUZIERUNG DER EMPFINDLICHKEIT VON NERVEN
STIMULATEUR PERCUTANÉ SERVANT À RÉDUIRE LA SENSIBILITÉ DE NERFS

(30) Priority: 11.07.2013 CN 201320412852 U
(43) Date of publication of application: 18.05.2016
(73) Proprietor: GiMer Medical Co., Ltd, New Taipei City 22175, Taiwan (TW)
(72) Inventor: LIN, Wei Tso, Taipei Taiwan (TW); WU, Chen Tun, Taipei Taiwan (TW); CHENG, Chan Yi, Taipei Taiwan (TW); CHANG, Chi Heng, Taipei Taiwan (TW)
(74) Representative: Zimmermann, Tankred Klaus
(86) International application number: PCT/CN2014/081169
(87) International publication number: WO 2015/003561

(56) References cited:
- WO-A1-96/20753
- WO-A1-2005/053790
- WO-A1-2009/064641
- CN-A- 1 048 501
- CN-U- 202 724 225
- CN-U- 203 469 232
- US-A1- 2005 222 635
- US-A1- 2013 116 742

## Description

### BACKGROUND

### Technical Field

The present invention is generally related to desensitizing devices, and more particular to a device to reduce the sensitivity of male genitals by providing low-strength stimulation.

### Related Art

In today's busy society, people's stress significantly affects their private lives. To make the sexual activity more fulfilling, a lot of sex toys are on the market to maintain and enhance people's interest and delight.

One common sex toy is the vibrator. However, a vibrator can only provide stimulation to the tactile sense, and has little effect on the male staying power, which is a major factor to a satisfying intercourse.

To promote male endurance, there are also various medications on the market. However, these medications often contain toxic or harmful components, and usually cause undesirable side effects.

WO 2009/064641 discloses an apparatus with an electrode disposed on a substrate and exposed at the bottom surface thereof for applying the at least nerve stimulating signal. A circuitized substrate includes a pair of electrodes accessible at the bottom surface thereof. An adhesive, conductive pad overlies the first electrode.

WO 96/20753 discloses an apparatus with a male electrode in the form of a ring to be wrapped around penile and/or scrotal tissue.

US 2013/116742 discloses an apparatus for measuring and treating the rigidity and erection of a penis and arterial-venous flows.

### SUMMARY

The invention is defined by independent claim 1. It refers to a desensitizing device for reducing the sensitivity of nerve through the skin and performing low-strength stimulation on the subcutaneous nerve so the subcutaneous nerve is temporarily numbed and desensitized.

By using the electrical stimulation member to perform low-strength stimulation on the subcutaneous nerve, the subcutaneous nerve is as such temporarily numbed and desensitized. For example, by configuring a male genital with the support member so that the electrodes are in contact with the penile skin, the stimulating current produced by the control circuit is conducted to the electrodes and provides a low-strength subcutaneous nerve stimulation through the penile skin. The subcutaneous nerve is as such temporarily numbed and desensitized, thereby enhancing the staying power of male genitals during intercourse. Since no medication is employed, potential undesirable side effects are avoided. In addition, the present invention can also be applied to other body parts (e.g., a wrist, an elbow, etc.). In exemplary, non-claimed, examples, the electrodes are attached directly to the desired body part. The stimulating current produced by the control circuit is conducted to the electrodes and provides a low-strength subcutaneous nerve stimulation through the skin of the desired body part. The subcutaneous nerve is as such temporarily numbed and desensitized so as to ease the neuralgia.

### BRIEF DESCRIPTION OF THE DRAWINGS

FIG. 1 is a perspective diagram showing a desensitizing device according to a first embodiment of the present invention.
FIG. 2 is a perspective break-down diagram showing the various components of the desensitizing device of FIG. 1.
FIG. 3 is a partial sectional diagram showing the desensitizing device of FIG. 1.
FIG. 4 is a partial sectional diagram showing the desensitizing device of FIG. 1.
FIG. 5 is a sectional diagram showing a desensitizing device according to a non-claimed example of the present invention.
FIG. 6 is a perspective diagram showing a desensitizing device according to a non-claimed example of the present invention.
Wherein:
1: support member
10: through channel
11: outer circumference
12: inner circumference
13: accommodation space
131: linear second opening
14: cover
15: linear opening
2: electrical stimulation member
21: control circuit
211: transmission wire
22: electrode
23: electric power supply unit
231: battery chamber
232: removable battery
24: switch
25: indicator lamp
3: belt-like support member

### DETAILED DESCRIPTION OF THE INVENTION

The following descriptions are exemplary embodiments only, and are not intended to limit the scope, applicability or configuration of the invention in any way. Rather, the following description provides a convenient illustration for implementing exemplary embodiments of the invention. Various changes to the described embodiments may be made in the function and arrangement of the elements described without departing from the scope of the invention as set forth in the appended claims.

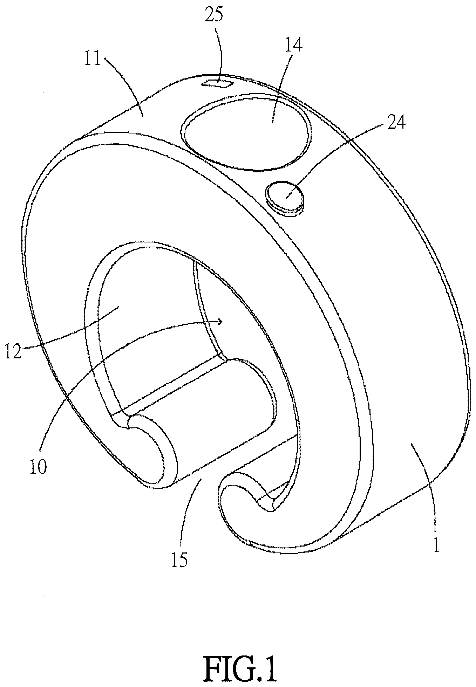

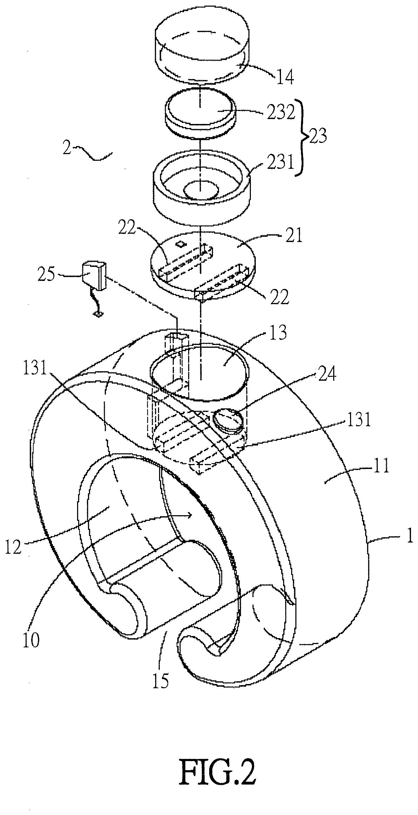

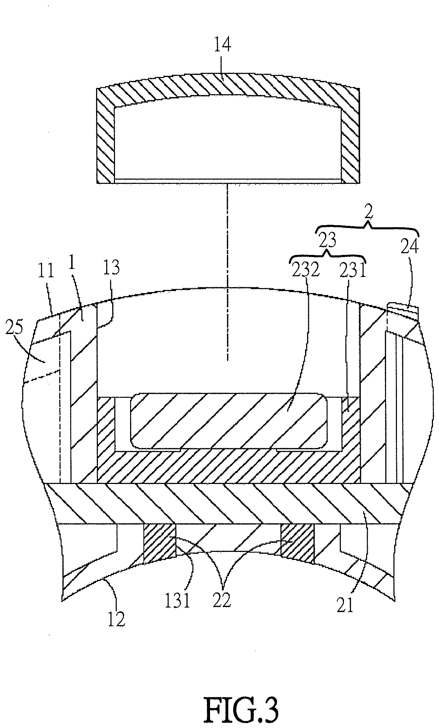

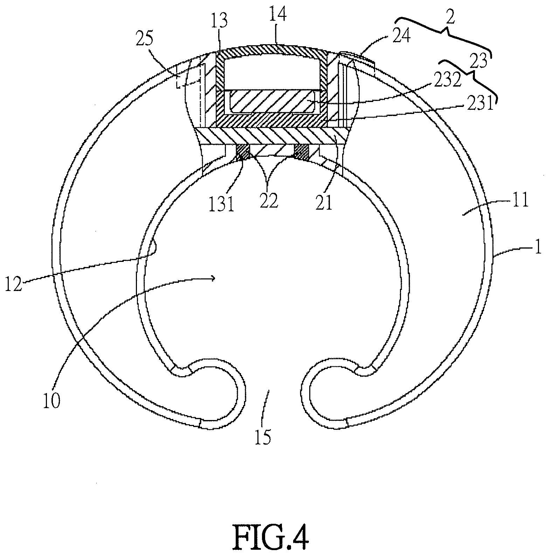

As shown in FIGS. 1 to 4, a desensitizing device according to a first embodiment of the present invention contains an electrical stimulation member 2 optionally configured on a support member 1. The support member 1 can be ring-shaped or in other appropriate shape.

The support member 1 has an outer circumference 11, an inner circumference 12, and a through channel 10 surrounded by the inner circumference 12. The support member 1 has an accommodation space 13 with a circular first opening on the outer circumference 11 sealed by a cover 14, and at least two linear second openings 131 on the inner circumference 12 so that the accommodation space 13 is connected to the through channel 10. The support member 1 is made of a flexible material and can be configured into a C-like shape with an axially-oriented linear opening 15 so that the linear opening 15 can be elastically broadened.

The electrical stimulation member 2 is housed in the accommodation space 13, and contains a control circuit 21, at least two electrodes 22, and an electric power supply unit 23. The electric power supply unit 23 can contain a removable battery or a built-in rechargeable battery, and is electrically connected to the control circuit 21 so as to provide electric power to the control circuit 21. In the present embodiment, the electric power supply unit 23 contains a removable battery 232 and a battery chamber 231. The battery chamber 231 has a positive terminal and a negative terminal, both connected to the control circuit 21. The removable battery 232 is accommodated in the battery chamber 231 and the removable battery 232's positive and negative terminals are connected to the battery chamber 231's positive and negative terminals, respectively. The at least two electrodes 22 are electrically connected to the control circuit 21, and are exposed to the through channel 10 on the inner circumference 12 through the second openings 131, respectively. With the electric power provided by the electric power supply unit 23, the control circuit 21 generates and delivers a stimulating current to the electrodes 22.

The stimulating current produced by the control circuit 21 can be a high-frequency signal between 200 KHz and 800 KHz, or a low-frequency signal between 0.5 Hz and 1 KHz (preferably between 2 Hz and 30 Hz), or a mixed signal with a high-frequency component and a low-frequency component. The high-frequency component is between 200 KHz and 800 KHz, and the low-frequency component is between 0.5 Hz and 1 KHz (preferably between 2 Hz and 30 Hz). The high-frequency or low frequency signal of the stimulating current can be a continuous wave or a train of intermittent pulses. The wave can be a sinusoidal wave, a triangular wave, a square wave, or one with an appropriately shaped waveform.

As the electrical stimulation member 2 is accommodated in the accommodation space 13, the control circuit 21 can be placed on a bottom side of the accommodation space 13. The electrodes 22 are electrically connected to a bottom side of the control circuit 21, configured in the second openings 131, respectively, and exposed from the inner circumference 12. The electric power supply unit 23 has the battery chamber 231 electrically connected to a top side of the control circuit 21, and the removable battery 232 is placed in the battery chamber 231. The first opening of the accommodation space 13 is then sealed by the cover 14 so that the electrical stimulation member 2 is tightly housed in the accommodation space 13.

The electrical stimulation member 2 further contains a switch 24 and an indicator lamp 25. The switch 24 is electrically connected to the control circuit 21 for turning the control circuit 21 on and off, and is exposed from the outer circumference 11 on the support member 1. The indicator lamp 25 is also electrically connected to the control circuit 21 and is exposed from the outer circumference 11 on the support member 1.

By threading a male genitals through the through channel 10 of the support member 1 so that the electrodes 22 are in contact with the penile skin, the stimulating current produced by the control circuit 21 when the electrical stimulation member 2 is turned on is conducted to the electrodes 22 and provides a low-strength subcutaneous nerve stimulation through the penile skin. The subcutaneous nerve is as such temporarily numbed and desensitized, thereby enhancing the staying power of male genitals during intercourse. Since no medication is employed, potential undesirable side effects are avoided.

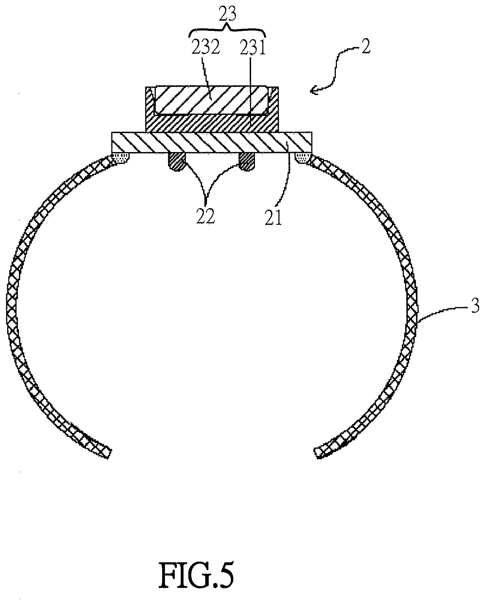

FIG. 5 depicts a a non-claimed example provided for better understanding of the present invention. As illustrated, a desensitizing device has an electrical stimulation member 2 configured on a belt-like support member 3. The support member 3 girdles a male genital so that the electrodes 22 of the electrical stimulation member 2 are in contact with the penile skin. The stimulating current produced by the control circuit 21 is conducted to the electrodes 22 and provides a low-strength subcutaneous nerve stimulation through the penile skin. The subcutaneous nerve is as such temporarily numbed and desensitized.

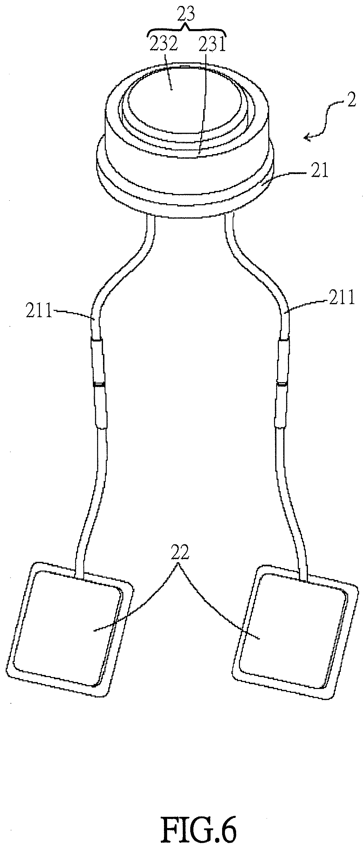

FIG. 6 depicts a non-claimed example provided for better understanding of the present invention. As illustrated, a desensitizing device has an electrical stimulation member 2 that is applied independently without a support member. The two electrodes 22 of the control circuit 21 are configured in two pads and extended by two transmission wires 211 so that the electrodes 22 can be attached to the penile skin directly. The stimulating current produced by the control circuit 21 is conducted to the electrodes 22 and provides a low-strength subcutaneous nerve stimulation through the penile skin. The subcutaneous nerve is as such temporarily numbed and desensitized.

In addition to providing low-strength subcutaneous nerve stimulation to male genitals, the high-frequency current produced by the control circuit 21 can also be applied to other body parts (e.g., a wrist, an elbow, etc.) for low-strength subcutaneous nerve stimulation. In these applications, the electrodes 22 are attached directly to the desired body part. The stimulating current produced by the control circuit 21 is conducted to the electrodes 22 and provides a low-strength subcutaneous nerve stimulation through the skin of the desired body part. The subcutaneous nerve is as such temporarily numbed and desensitized so as to ease the neuralgia.

## Claims

1. A desensitizing device, comprising an electrical stimulation member (2) and a support member (1), the support member (1) having an outer circumference (11), an inner circumference (12), a through channel (10) surrounded by the inner circumference (11), and an accommodation space (13) with a first opening on the outer circumference (11) and at least two second openings (131) on the inner circumference (12), wherein the electrical stimulation member (2) is disposed in the support member (1) and comprises:
a control circuit (21) configured for producing a stimulating current which comprises a first frequency signal between 200 KHz and 800 KHz;
at least two electrodes (22) exposed from the inner circumference (12) through the second openings (131), respectively, and configured to be attached to a user's skin and electrically connected to the control circuit (21), configured for receiving the simulating current from the control circuit (21), and for applying the stimulating current to the skin, so that the stimulating current is provided to a subcutaneous nerve through the skin, and the subcutaneous nerve is temporarily desensitized; and
an electric power supply unit (23) electrically connected to the control circuit (21) for providing electric power to the control circuit (21).

2. The desensitizing device according to claim 1, wherein the stimulating current further comprises a second frequency signal between 0.5 Hz and 1 KHz.

3. The desensitizing device according to one of claims 1-2, wherein the first frequency signal of the stimulating current is a continuous wave.

4. The desensitizing device according to one of claims 1-2, wherein the first frequency signal of the stimulating current is a train of intermittent pulses.

5. The desensitizing device according to claim 2, wherein the second frequency signal of the stimulating current is between 2 Hz and 30 Hz.

6. The desensitizing device according to claim 2, wherein the second frequency signal of the stimulating current is a continuous wave.

7. The desensitizing device according to claim 2, wherein the second frequency signal of the stimulating current is a train of intermittent pulses.

8. The desensitizing device according to one of claims 1-2, wherein the support member (1) made of a flexible material and configured into a substantially ring-like shape.

9. The desensitizing device according to one of claims 1-2, wherein the desensitizing device is configured on a support member (1), and the support member (1) is belt-shaped.

10. The desensitizing device according to one of claims 1-2, wherein the electrodes (22) are extended from the control circuit (21) by a plurality of transmission wires (211), respectively.

11. The desensitizing device according to one of claims 1-2, wherein the electric power supply unit (23) comprises a removable battery (232).

12. The desensitizing device according to one of claims 1-2, wherein the electric power supply unit (23) comprises a built-in rechargeable battery.

13. The desensitizing device according to claim 8, wherein the electrical stimulation member (2) is configured on the support member (1).

## Patentansprüche

1. Desensibilisierungsvorrichtung, umfassend ein elektrisches Stimulationselement (2) und ein Trägerelement (1), wobei das Trägerelement (1) einen Außenumfang (11), einen Innenumfang (12), einen Durchgangskanal (10), der durch den Innenumfang (11) umgeben ist, und einen Aufnahmeraum(13) mit einer ersten Öffnung an dem Außenumfang (11) und wenigstens zwei zweiten Öffnungen (131) an dem Innenumfang (12) aufweist, wobei das elektrische Stimulationselement (2) in dem Trägerelement (1) angeordnet ist und umfasst:
eine Steuerschaltung (21), die zum Erzeugen eines Stimulationsstroms konfiguriert ist, der ein erstes Frequenzsignal zwischen 200 KHz und 800 KHz umfasst;
wenigstens zwei Elektroden (22), die jeweils durch die zweiten Öffnungen (131) aus dem Innenumfang (12) freiliegen und dazu konfiguriert sind, an der Haut eines Benutzers angebracht und elektrisch mit der Steuerschaltung (21) verbunden zu sein, dazu konfiguriert sind, den Strom von der Steuerschaltung (21) aufzunehmen, und dazu, den Stimulationsstrom an die Haut anzulegen, sodass der Stimulationsstrom durch die Haut an einen subkutanen Nerv bereitgestellt wird und der subkutane Nerv vorübergehend desensibilisiert wird; und
eine Stromversorgungseinheit (23), die elektrisch mit der Steuerschaltung (21) verbunden ist, um elektrischen Strom an die Steuerschaltung (21) bereitzustellen.

2. Desensibilisierungsvorrichtung nach Anspruch 1, wobei der Stimulationsstrom ferner ein zweites Frequenzsignal zwischen 0,5 Hz und 1 KHz umfasst.

3. Desensibilisierungsvorrichtung nach einem der Ansprüche 1-2, wobei das erste Frequenzsignal des Stimulationsstroms eine kontinuierliche Welle ist.

4. Desensibilisierungsvorrichtung nach einem der Ansprüche 1-2, wobei das erste Frequenzsignal des Stimulationsstroms eine Folge von intermittierenden Impulsen ist.

5. Desensibilisierungsvorrichtung nach Anspruch 2, wobei das zweite Frequenzsignal des Stimulationsstroms zwischen 2 Hz und 30 Hz beträgt.

6. Desensibilisierungsvorrichtung nach Anspruch 2, wobei das zweite Frequenzsignal des Stimulationsstroms eine kontinuierliche Welle ist.

7. Desensibilisierungsvorrichtung nach Anspruch 2, wobei das zweite Frequenzsignal des Stimulationsstroms eine Folge von intermittierenden Impulsen ist.

8. Desensibilisierungsvorrichtung nach einem der Ansprüche 1-2, wobei das Trägerelement (1) aus einem flexiblen Material hergestellt und in Wesentlichen in einer ringartigen Form konfiguriert ist.

9. Desensibilisierungsvorrichtung nach einem der Ansprüche 1-2, wobei die Desensibilisierungsvorrichtung an einem Trägerelement (1) konfiguriert ist und das Trägerelement (1) riemenförmig ist.

10. Desensibilisierungsvorrichtung nach einem der Ansprüche 1-2, wobei die Elektroden (22) sich jeweils durch eine Vielzahl von Übertragungsdrähten (211) von der Steuerschaltung (21) erstrecken.

11. Desensibilisierungsvorrichtung nach einem der Ansprüche 1-2, wobei die Stromversorgungseinheit (23) eine herausnehmbare Batterie (232) umfasst.

12. Desensibilisierungsvorrichtung nach einem der Ansprüche 1-2, wobei die Stromversorgungseinheit (23) eine eingebaute wiederaufladbare Batterie umfasst.

13. Desensibilisierungsvorrichtung nach Anspruch 8, wobei das elektrische Stimulationselement (2) an dem Trägerelement (1) konfiguriert ist.

## Revendications

1. Appareil de désensibilisation, comprenant un élément de stimulation électrique (2) et un élément de support (1), l'élément de support (1) ayant une circonférence extérieure (11), une circonférence intérieure (12), un canal traversant (10) entouré par la circonférence intérieure (11), et un espace de logement (13) avec une première ouverture sur la circonférence extérieure (11) et au moins deux deuxièmes ouvertures (131) sur la circonférence intérieure (12), dans lequel l'élément de stimulation électrique (2) est disposé dans l'élément de support (1) et comprend :
un circuit de commande (21) configuré pour produire un courant stimulant qui comprend un premier signal de fréquence entre 200 KHz et 800 KHz ;
au moins deux électrodes (22) exposées depuis la circonférence intérieure (12) à travers les deuxièmes ouvertures (131), respectivement, et configurées pour être fixées à la peau d'un utilisateur et raccordées électriquement au circuit de commande (21), configurées pour recevoir le courant stimulant depuis le circuit de commande (21), et pour appliquer le courant stimulant sur la peau, de sorte que le courant stimulant soit fourni à un nerf sous-cutané à travers la peau, et le nerf sous-cutané soit temporairement désensibilisé ;
et une unité d'alimentation électrique (23) raccordée électriquement au circuit de commande (21) pour fournir l'énergie électrique au circuit de commande (21).

2. Appareil de désensibilisation selon la revendication 1, dans lequel le courant stimulant comprend en outre un deuxième signal de fréquence entre 0,5 Hz et 1 KHz.

3. Appareil de désensibilisation selon l'une des revendications 1 et 2, dans lequel le premier signal de fréquence du courant stimulant est une onde continue.

4. Appareil de désensibilisation selon l'une des revendications 1 et 2, dans lequel le premier signal de fréquence du courant stimulant est un train d'impulsions intermittentes.

5. Appareil de désensibilisation selon la revendication 2, dans lequel le deuxième signal de fréquence du courant stimulant est entre 2 Hz et 30 Hz.

6. Appareil de désensibilisation selon la revendication 2, dans lequel le deuxième signal de fréquence du courant stimulant est une onde continue.

7. Appareil de désensibilisation selon la revendication 2, dans lequel le deuxième signal de fréquence du courant stimulant est un train d'impulsions intermittentes.

8. Appareil de désensibilisation selon l'une des revendications 1 et 2, dans lequel l'élément de support (1) est constitué d'un matériau flexible et sensiblement de forme annulaire.

9. Appareil de désensibilisation selon l'une des revendications 1 et 2, dans lequel l'appareil de désensibilisation est configuré sur un élément de support (1), et l'élément de support (1) est en forme de courroie.

10. Appareil de désensibilisation selon l'une des revendications 1 et 2, dans lequel les électrodes (22) sont étendues depuis le circuit de commande (21) par une pluralité de fils de transmission (211), respectivement.

11. Appareil de désensibilisation selon l'une des revendications 1 et 2, dans lequel l'unité d'alimentation électrique (23) comprend une batterie amovible (232).

12. Appareil de désensibilisation selon l'une des revendications 1 et 2, dans lequel l'unité d'alimentation électrique (23) comprend une batterie rechargeable intégrée.

13. Appareil de désensibilisation selon la revendication 8, dans lequel l'élément de stimulation électrique (2) est configuré sur l'élément de support (1).
